# EUROPEAN PATENT SPECIFICATION

(11) **EP 1 392 290 B1**
(45) Date of publication and mention of the grant of the patent: **18.05.2005**
(21) Application number: 02730033.4
(22) Date of filing: 20.03.2002
(51) Int. Cl.: A61K 31/4015, A61P 31/04, A61P 31/12, A61P 33/00, A61P 31/16, A61P 31/18, A61P 35/00

(54) **USE OF CERTAIN SUBSTITUTED PYRROLIDONES SUCH AS PIRACETAM IN THE TREATMENT OF VIRAL AND OTHER DISEASES**
VERWENDUNG BESTIMMTER SUBSTITUIERTER PYRROLIDONE WIE Z.B. PIRACETAM BEI DER BEHANDLUNG VON VIRALEN UND ANDEREN ERKRANKUNGEN
UTILISATION DE CERTAINES PYRROLIDONES SUBSTITUEES TELLES QUE LA PIRACETAME DANS LE TRAITEMENT DE MALADIES VIRALES ET AUTRES AFFECTIONS

(30) Priority: 22.03.2001 EP 01107140
(43) Date of publication of application: 03.03.2004
(73) Proprietor: UCB, S.A., 1070 Bruxelles (BE)
(72) Inventor: PEUVOT, Jacques, B-1470 Bousval (BE); BRASSEUR, Robert, B-5351 Haillot (BE); DELEERS, Michel, B-1630 Linkebeek (BE); PONTES, Fausto A., Centro Hospitalar de Coimbra, Coimbra (PT); RUYSSCHAERT, Jean-Marie, B-1640 Rhode St. Genèse (BE)
(74) Representative: Lechien, Monique
(86) International application number: PCT/EP2002/003106
(87) International publication number: WO 2002/076451

(56) References cited:
- EP-A- 0 165 919
- US-A- 4 145 347
- NALBANDIAN R M ET AL: "Erythrocyte - endothelial cell adherence in sickle cell disease, diabetes mellitus, and falciparum malaria: adverse effects reversed with piracetam." MEDICAL HYPOTHESES, (1982 FEB) 8 (2) 155-62., XP001027520
- ROSSINI P M ET AL: "Jakob- Creutzfeldt disease: analysis of EEG and evoked potential under basal conditions and neuroactive drugs." EUROPEAN NEUROLOGY, (1979) 18 (4) 269-79., XP002188464
- CEREGHINO J J ET AL: "Levetiracetam for partial seizures: Results of a double-blind, randomized clinical trial." NEUROLOGY, vol. 55, no. 2, 25 July 2000 (2000-07-25), pages 236-242, XP002188465 ISSN: 0028-3878

## Description

The present invention concerns the use of certain substituted pyrrolidones, their phamaceutically acceptable salts and their isomers in the prevention and treatment of viral diseases resulting from tilted insertion of fusion proteins into the membranes of cells and for the preparation of medicaments intended for the prevention and treatment of such diseases.

Cellular life exists because of a segregation between the interior of the cell and its external environment. This separation is achieved by a biological membrane which is essentially formed of lipids and proteins. The lipid composition and the protein composition vary considerably from one cellular type to another. However, in all cases, the membrane of the cell is a barrier which prevents prolonged contact between the intracellular domain and the extracellular liquid.

Nevertheless a number of phenomena are involved in the re-organisation of membrane structure, for example to allow the fusion and passage of proteins across the membrane. Membrane fusion reactions occur continuously in all eukaryotic cells. and are involved in different processes such as endocytosis, exocytosis, and enveloped virus infections which require one or more critical membrane fusion reactions.

In particular, the entry into the cell of many infectious agents such as viruses, parasites and bacterial pathogens proceeds *via* a mechanism of fusion and passage of proteins across the membrane.

A key feature in viral and cellular fusion phenomena is the involvement of specific fusion proteins. Among the few well-characterized fusion proteins are the viral spike glycoproteins which are responsible for penetration of enveloped viruses into their host cells leading to infection.

These proteins possess in their sequence a "fusion peptide", a short segment, typically no more than 40 especially up to 20 amino acids long, of relatively hydrophobic residues, commonly found in a membrane-anchored polypeptide chain.

Conformational analysis of the fusion protein of the virus causing Newcastle disease has made possible an understanding of its mode of action (Le *et al*., 1988 Virus Genes 1 (4), 333-350). Classical sequence analysis methods have revealed that the N-terminal portion of the fusion protein is strongly hydrophobic and molecular modeling has shown that this peptide inserts itself in oblique or tilted fashion in the lipids.

In recent years, candidate virus-cell fusion proteins involved in different fusion processes have been identified, and evidence concerning the presence of a fusion peptide has been obtained for some of these proteins (Muga *et al*., 1994 Biochemistry 33, 3201-3209; Hernandez *et al*., 1996 Annu. Rev. Cell Dev. Biol. 12, 627-661; Wolfsberg and White, 1996 Dev. Biol., 180, 389-40; Martin and Ruysschaert, 1997 FEBS Lett 405, 351-355; Schanck *et al*., 1998 J.Chim Phys. 95, 467-473).

Other viral fusion peptides, including that of SIV, have been analysed by molecular modeling and were shown to possess the property of inserting themselves obliquely as α-helices within the lipid layers

Candidate fusion proteins have also been evidenced by conformational analysis of the fusion proteins of parasites.

For malaria, affecting about 280 millions of people worldwide, the CS (circumsporozoite) protein located at the surface of the sporozoite membrane has been recognized to be responsible for the binding and the penetration of the malarial parasite in hepatocytes (Cerami *et al*. 1992 Cell 70, 1021-1033; Ménard *et al*., 1997 Nature 385, 336-340).

Molecular modeling has identified an amino acid fragment in this protein exhibiting the properties of a potential fusion peptide that inserts obliquely with a tilted insertion angle of 50° into the lipid layers (Peuvot *et al*., 1999, J. Theor.Biol. 198, 173-181).

When bacterial infections are considered, an important step in the understanding of infective mechanisms by the tuberculosis bacillus was made in 1993 (Arruda *et al*., 1993 Science 261, 1454-1457). *Mycobacterium tuberculosis* first infects macrophages. A DNA fragment was isolated that allows the bacillus to enter the macrophage and to survive in human cells. This fragment was introduced into the genome of *E.coli* which was then able to penetrate inside macrophages and to resist destruction. This DNA fragment contains two sub-units responsible, respectively, for penetration and survival in the cell.

The occurrence of tilted peptides has been discovered in two neurodegenerative diseases. The discovery of these tilted fragments in proteins involved in these neurodegenerative diseases originally surprised researchers, since those peptides were not known to be involved in membrane fusion (Brasseur et *al*., 1997 TIBS 22, 167-171).

Alzheimer's disease (AD) is a progressive dementia affecting about 5 - 10 % of people over the age of 65 (Ebly *et al*., 1994; Neurology 44, 1593-1600). AD is characterized pathologically by the deposition of extracellular amyloid, containing the β -amyloid protein (Aβ) which is derived from the β-amyloid protein precursor (APP) (Kang *et al*., 1987 Nature 325, 733-736; Glenner and Whong, 1994 Biochem. Biophys. Res. Commmun. 120, 885-890; Masters *et al*., 1985 Proc. Natl. Acad. Sci. USA 82, 4245-4249).

Computer modeling of the β-amyloid peptide showed that the C-terminal domain of Aβ (e.g. 29-40 or 29-42 peptide) has properties similar to those of the fusion peptide of viral proteins (Horth *et al*., 1991 EMBO J. 10, 2747-2755).

The 29-42 tilted peptide inserts into the membrane with an insertion angle of 50° and the 29-40 peptide with an insertion angle of 40°. Aβ-(29-42) peptide is the more potent fusogenic peptide and its fusogenic properties are closest to those of the SIV fusion peptide, because it can insert deeper into the lipid bilayer than the Aβ-(29-40) peptide (Pillot *et al*., 1996 J.Biol.Chem. 271, 28757-28765).

Several proteins involved in other neurodegenerative diseases were also analyzed and all have tilted peptides.

Therefore, whatever the particular mechanism may be, tilted peptides could play a role in neurodegenerative diseases.

Brasseur, 1991 (J. Biol. Chem. 266, 16120-16127), Peuvot et al. 1999 (J. Theor.Biol. 198, 173-181) and Brasseur et al. 2000 (Mol. Membrane Biol. 17(1), 31-40) provide an overview of the above-mentioned studies and others and include examples of viral fusion proteins and methods for their detection.

Thus, tilted peptides are found in many fragments of proteins previously described to experimentally induce fusion and it appears that the tilted insertion of fusion peptides plays a crucial role in the development of many different diseases including viral, parasital, bacterial, cancerous and neurodegenerative diseases.

Therefore, a means of interfering with these peptide functions involving tilted insertion of fusion peptides would present considerable therapeutic interest in creating for example antiviral, antiparasital, antibacterial, anticancerous, or antineurodegenerative agents. It is a purpose of the present invention to provide such a means.

A study using the complementary combination of an experimental tool, the ³¹P NMR, and a theoretical method, conformational analysis, has described the molecular effect of the substituted pyrrolidone, piracetam (Peuvot *et al*., 1995 Biochem. Pharmacol 50-8, 1129-1134). An *in vitro* test has also shown the ability of piracetam to stop the replication of herpes simplex virus 1 (HSV1) in Vero cells culture (Y.M. Centifanto, 1992 Antiviral Res. 17, suppl. 1, 114).

It has now surprisingly been found that certain substituted pyrrolidones are able to prevent or inhibit destabilisation induced by the tilted insertion of fusion peptides into the membranes of cells.

The present invention therefore concerns the use of a compound of formula I, in racemic or isomeric form, or a pharmaceutically acceptable salt thereof, 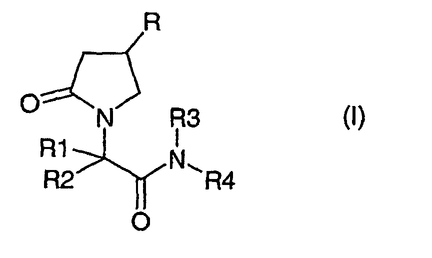 wherein
- R represents hydrogen or hydroxy;
- R¹ and R² represent independently hydrogen or an alkyl group of 1-4 carbon atoms; and
- R³ and R⁴ represent independently hydrogen, an alkyl group of 1-4 carbon atoms or -(CH₂)ₙ-NR⁵R⁶ wherein n is 1, 2 or 3 and R⁵ and R⁶ represent independently hydrogen or an alkyl group of 1-4 carbon atoms;
for the preparation of a medicament to prevent and treat diseases resulting from or mediated by the tilted insertion of fusion peptides into cell membranes.

The compound of formula I wherein R, R¹, R², R³ and R⁴ are hydrogen, 2-oxo-pyrrolidineacetamide, has the generic name PIRACETAM has been described in UK Patents Nos. 1,039,113 and 1,309,692 and is useful in the treatment of motion sickness, hyperkinesia and epilepsy.

UK Patent No. 1,309,692 also describes the compound of formula I wherein R¹ is ethyl, and R, R², R³ and R⁴ are hydrogen, in racemic form, α-ethyl-2-oxo-1-pyrrolidineacetamide, also known as ETIRACETAM.

The compound of formula I wherein R¹ is ethyl, and R, R², R³ and R⁴ are hydrogen, in levorotatory form, (S)-α-ethyl-2-oxo-1-pyrrolidineacetamide, also known as LEVETIRACETAM, has been described in EP 0 162 036 B1 as a drug useful in preventing hypoxia and ischemia.

LEVETIRACETAM is also an efficacious therapeutic agent for the treatment of epilepsy (Gower *et al*., 1992 Eur. J. Pharmacol. 222, 2-3).

The compound of formula I wherein R¹ is an ethyl, and R , R², R³ and R⁴ are hydrogen, in dextrorotatory form, (R)-(-) alpha-ethyl-2-oxo-1-pyrrolidineacetamide, has been described in EP 0 165 919 B1 as a drug useful to treat cerebral insufficiencies and memory disorders.

The compound of formula I wherein R, R¹, R² and R³ are hydrogen and R⁴ is diisopropylaminoethyl, N-[2-(diisopropylamino)ethyl]-2-oxo-1-pyrrolidineacetamide, also known as PRAMIRACETAM, has been described in US Patent No. 4,145,347 along with its use as a cognition activator and in treating induced amnesia and learning disabilities.

The compound of formula I wherein R is hydroxy and R¹, R², R³ and R⁴ are hydrogen, 4-hydroxy-2-oxo-1-pyrrolidineacetamide, also known as OXIRACETAM, has been described in US Patent No. 4,118,396 along with its CNS activity.

The remaining compounds of formula I are new and also form part of the invention.

The compounds for use in accordance with the present invention are able to inhibit the tilted insertion of fusion peptides into the membranes of the cells. This makes them particularly useful to treat or preferably prevent diseases resulting from or mediated by the tilted insertion of fusion peptides into cell membranes.

As discussed herein a wide variety of diseases appear to be mediated by the tilted insertion of fusion proteins into the membranes of cells. Non-limiting examples include viral diseases, such as Newcastle disease, bovine leukemia, AIDS, influenza, measles, rubella, hepatitis B, hepatitis C and Ebola fever; parasitic infestations such as malaria or the disease provoked by *Trypanosona cruzi;* bacterial infections such as provoked by *Mycobacterium tuberculosis*, by *Yersinia pseudotuberculosis* or by *Listeria monocytogenes*, conditions leading to the development of cancerous cells or development of neurodegenerative diseases such as Alzheimer's disease or Creutzfeldt-Jakob disease (including the new variant).

The uses according to the invention are particularly suited for the treatment & prevention of viral diseases especially Newcastle disease, bovine leukemia, Aids, influenza, measles, rubella, hepatitis B, hepatitis C and Ebola fever.

As the nature of the effect exhibited by the compounds of formula I is thought to be physical and/or mechanical it has the advantage that it is likely to be nonspecific with respect to particular diseases and also less likely to be prone to common drug-resistance and/or drug-interaction phenomena. The preferred compounds of formula I for use in accordance with the present invention are those wherein R², R³ and R⁴ are hydrogen and R¹ is hydrogen or straight chain alkyl of 1-4 carbon atoms.

Particularly preferred compounds for use in accordance with the present invention are piracetam, levetiracetam and (R)-(-)-alpha-ethyl-2-oxo-1-pyrrolidineacetamide.

The term "pharmaceutically acceptable salt" as used herein means addition salts with pharmaceutically acceptable non-toxic organic and inorganic acids, such as acetic, citric, maleic, succinic, ascorbic, hydrochloric, hydrobromic, sulfuric, and phosphoric acids and the like.

The term " isomer" as used herein means, when the molecule has a center of asymmetry, the levorotatory and the dextrorotatary enantiomers thereof, more precisely, it means that the said compound comprises at least 90%, preferably at least 95%, by weight of the said individual (either dextro- or levorotatory) optical isomer and at most 10%, preferably at most 5% by weight of the other individual (respectively levo- or dextrorotatary) optical isomer. Each individual optical isomer may be obtained by conventional means, i.e., resolution from the corresponding racemic mixture or asymmetric synthesis.

The term "fusion peptide" as used herein has the meaning understood in the art namely a short segment of no more than 40 amino acids in length of relatively hydrophobic/hydrophilic residues found in a protein, commonly in a membrane anchored or interacting polypeptide chain and involved in destabilising cell membranes.

The term "tilted insertion" means insertion into the membrane of the cell across the water/lipid (hydrophilic/hydrophobic) interface at an oblique angle usually between 25° and 75°, particularly 45° and 65°, especially 50° and 60° between to the plane of the cell membrane containing the interface.

Determining whether a particular disease or condition is mediated by or results from the tilted invention of fusion peptides into cell membranes can be determined for example using the methodologies described and reviewed in Brasseur 1991 and Peuvot 1999 referred to above, the content of each of which in this respect is incorporated herein by reference . Once this determination has been made conventional *in vivo* and *in vitro* testing will establish effectiveness of compounds. Additionally, the likely suitability of a particular compound for interaction with a particular peptide to affect the tilted insertion of fusion peptide can be determined for example in accordance with Peuvot 1995 referred to above and incorporated herein by reference

For implementing use according to the invention, a therapeutically effective amount of a compound of formula I in racemic or isomeric form, or a pharmaceutically acceptable salt thereof should be administered to the patient. An effective amount can be readily determined by the use of conventional techniques and by observing results obtained under analogous circumstances. In determining the effective amount, a number of factors are considered including, but not limited to: the species of patient; his/her body weight, age, and general health; the specific disease to be treated or prevented; the degree of or involvement or the severity of the disease; the response of the individual patient; the particular compound administered; the mode of administration; the bioavailability characteristics of the preparation administered: the dose regimen selected; and the use of concomitant medication.

As stated above, in accordance with the present invention the compounds of formula I in racemic or isomeric form, or as pharmaceutically acceptable salts thereof may be used to manage viral diseases resulting from the tilted insertion of fusion peptides into cell membranes. This can be done by preventing onset of the disease or by treating it in its acute or chronic form.

For the preventative treatment, the daily dosage can be low, however intake of the drug has to start as early as possible (CITIUS) to prevent the appearance of the disease.

Compliance has to be strictly maintained, as long as the threat of the disease lasts. Any missing of drug intake will allow fusion peptides of pathological proteins to insert obliquely into the membranes of the uninfected cells, subsequently leading to the appearance of the clinical stage of the disease, since the synthesis of fusion peptides by infected cells, not stopped by the drug, will continue.

For treatment of the acute and chronic stages of the disease, the daily dosage should be higher (ALTIUS). Drug intake has to start as early as possible (CITIUS), once diagnosis has been made, to decrease evolution of the disease, to arrest it, and if possible to eradicate it as could be the case for some diseases such as viral diseases.

This favorable effect may be achieved by the efficacy of the compounds according to the invention, as monotherapy, or as add-on therapy to the most efficient products available for the particular disease [for example existing tritherapy / quadritherapy in the case of AIDS (FORTIUS)] in addition to the efficacy of the immune system if still active.

Drug intake has to be strictly maintained for as long as clinical and biological examination of the patient shows continued existence of the acute or chronic stage of the disease.

Any missing of drug intake will allow fusion peptides of pathological proteins to insert obliquely into the membranes of the uninfected cells. This in turn will lead to the maintenance of the acute clinical stage of the disease eventually allowing the passage to the chronic stage or the maintenance of a pre-existing chronic stage since the synthesis of fusion peptides by infected cells, not stopped by the drug, will continue.

For administration in accordance with the present invention a compound of formula I in racemic or isomeric form, or a pharmaceutically acceptable salt thereof may be got up in the form of pharmaceutical compositions which may be administered by any conventional means.

Pharmaceutical compositions intended for oral administration may be solid or liquid, for example, in the form of tablets, pills, dragees, gelatin capsules, sublingual formulations, solutions, syrups, and the like.

For this purpose, the active compounds can be mixed with an inert diluent or a pharmaceutically acceptable non-toxic carrier, such as for example starch or lactose. Optionally, these pharmaceutical compositions can also contain a binder such as microcrystalline cellulose, gum tragacanth or gelatin, a disintegrating agent such as alginic acid, a lubricant such as magnesium stearate, a glidant such as colloidal silicon dioxide, a sweetening agent such as sucrose or saccharin, a coloring agent or a flavoring agent such as peppermint or methyl salicylate. These compositions also include compositions that allow controlled release of the active substance.

Other modes of administration include by injection either intravenously, subcutaneously or intramuscularly.

All such pharmaceutical forms may be prepared according to conventional methods used by pharmacists.

The percentage of active compound in the pharmaceutical compositions can vary within very wide concentration limits and depends on a variety of factors such as the sex, age, weight and medical condition of the patient, as well as the mode of administration.

In the preferred oral compositions, the dosage unit contains between 250 and 1500mg of active compound. For example in the case of PIRACETAM dosage units containing 400, 800 or 1200mg of active ingredient are appropriate.

As regards the daily dosage, this can vary within a wide range of dosage units and it is to be understood that the specific dose will be adapted for particular cases, depending on individual need, at the discretion of the responsible physician. However, for evident pharmacokinetic reasons and due to the mechanism of action of the compounds of the present invention by blocking the fusion of tilted peptides on the membranes of the host cells, it is recommended that the compounds are administered in three divided daily doses.

The following illustrative dosages for piracetam are given for exemplary purposes only and by no means limit the scope of practice of the invention.

As an example, for piracetam, the recommended oral dose schedule for preventative treatment is from 2.4 g to 4.8 g daily.

For acute phase treatment, the recommended oral dose schedule is from 4.8 g daily up to 10.8 g daily depending on the disease and on the severity of the clinical state (for example HBV, HCV, AIDS).

For long term treatment of chronic diseases (for example HBV, HCV, AIDS) the recommended oral dose schedule is from 4.8 g daily up to 10.8 g daily.

In severe cases, when patients have already an intravenous access, large doses of up to 12 g daily of piracetam may be administered, also in three divided daily doses.

The following compositions are examples of compositions, containing compounds for use according to the invention, suitable for oral administration:

| Composition 1 | Composition 2 |
|---|---|
| XX mg piracetam | XX mg levetiracetam |
| 200 mg lactose | 400 mg lactose |
| 1 mg magnesium stearate | 5 mg magnesium stearate |

The invention therefore concerns the use of a compound of formula I, in racemic or isomeric form, or a pharmaceutically acceptable salt thereof, 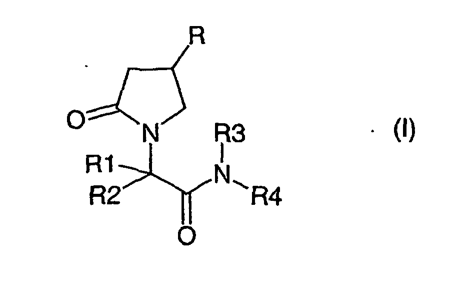 wherein
- R represents hydrogen or hydroxy;
- R¹ and R² represent independently hydrogen or an alkyl group of 1-4 carbon atoms; and
- R³ and R⁴ represent independently hydrogen, an alkyl group of 1-4 carbon atoms or -(CH₂)ₙ-NR⁵R⁶ wherein n is 1, 2 or 3 and R⁵ and R⁶ represent independently hydrogen or an alkyl group of 1-4 carbon atoms; for the preparation of a medicament to prevent and treat viral disease.

Particular viral diseases susceptible to such prevention & treatment include Newcastle disease, bovine leukemia, HIV, AIDS, influenza, measles, rubella, hepatitis B, hepatitis C, ebola fever.

The invention is further defined by reference to the following examples describing the efficiency of methods of treatment and prevention according to the present invention.

### EXAMPLE 1. Effect of piracetam on patients exhibiting acute viral hepatitis.

### Study design

110 patients with acute hepatitis, hospitalised from the first or the second week of icterus, were included in the study. All patients were assigned to rest, and received diet and vitamins.

A first group received piracetam at a daily dose of 2.4 g (3 x 0.8 g/day), a second group received no piracetam and acted as control group. The main parameters were serum bilirubine and transaminases, measured once a week during the whole icterus period.

Inclusion criteria were bilirubine (>3.0 mg/daily) (N<0.3) and transaminases (>400 U) (N<40).

The criteria for a positive response were bilirubine <1.5 mg/dl and transaminases <100 U.

### Results

Clinical tolerance was excellent.

The evolution in serum bilirubine levels was very highly significantly reduced (p < 0.0003) for the piracetam group when compared to the control group. This positive evolution was especially observed for patients receiving piracetam from the first week of icterus (time effect) while bulirubine levels were not significantly reduced in patients receiving piracetam from the second week of icterus.

The evolution in serum transaminases was also favourable, although non-significant, in patients receiving piracetam. Again, this effect appeared more pronounced for patients receiving piracetam from the first week of icterus suggesting a time-effect relationship.

Except for 6 patients out of 49, the icterus phase for the piracetam group was reduced by 50% when compared to the control group.

These results indicate a potential therapeutic effect of piracetam in acute hepatitis.

### EXAMPLE 2. Effect of piracetam on patients exhibiting chronic C virus hepatitis.

### Study design

6 patients with chronic transfusion associated hepatitis C virus (HCV) received 6 months therapy with piracetam at a daily dose of 10.8 g (3 x 3.6 g/d) with no other treatment associated. This was followed by a 3 months period of no treatment.

All patients, interferon non-responders, had chronic hepatitis from 6 to 19 years. The main criterion of efficacy was serum viral load (HCV-RNA) performed by polymerase chain reaction (PCR) at 0, 3, 6 and 9 months.

These patients had manifested a gradual rise in their HCV-RNA. Thus, the criteria for no response was defined as no change in the increase of HCV-RNA, a positive response as a *status quo* of serum HVC-RNA and a significant response as a decrease of at least half a log of HCV-RNA.

### Results

Clinical tolerance was excellent

Two patients had an increase inferior to 10%. Four patients had a decrease, which was significant (↘ < 0.6 log) in the patient who was the most recently infected (6 years) suggesting potential therapeutic effect at piracetam in chronic hepatitis C.

### EXAMPLE 3. In vitro inhibitory effect of piracetam on the formation of syncitia induced by fusion of HIV infected cells with uninfected neighbouring cells.

HIV has the property on cultured cells to provoke the formation of syncitia by fusion of infected cells with uninfected neighboring cells, when the virus ends its replication.

Virus have become remarkably opportunistic in exploring whatever complicated shapes they may find on the host cell surface as binding receptors (e.g. CD4, CXCR4, CCR5, CKR-5, CKR-3).

This study therefore investigated the ability of piracetam to inhibit the formation of syncitia, while interfering with CD4, the primary binding receptor of HIV. For this purpose, two cultures of cells were used, A-201 cells devoid of CD4 receptors and SUP-T1 cells presenting CD4 receptors.

Piracetam, accordingly to the doses used, inhibited the process leading to the formation of syncitia, whether or not the cell presents the CD4 binding receptor, confirming its aspecific action. This in vitro effect on the membrane is very clear.

Based upon this result, the ability of piracetam to inhibit the fusion of large unilamellar vesicles (LUV), made of the main 4 PL of the envelope of the virus, and provoked by the titled peptide of HIV-wt (wild type)(16 AA) and of SIV-wt (12AA) was investigated.

At the doses tested, piracetam inhibits the fusion of LUV in the presence of these two tilted peptides.

### EXAMPLE 4. Levetiracetam and (R)-(-)-alpha-ethyl-2-oxo-1-pyrrolidineacetamide.

The same in vitro study as in Example 1 was used to investigate two analogues (levetiracetam and (R)-(-)-alpha-ethyl-2-oxo-1-pyrrolidine acetamide) in comparison with piracetam.
At the doses tested, these two analogues have also shown a very clear inhibitory effect on the fusion process induced on LUV by the viral tilted peptide.

### Reference

### EXAMPLE 5. In vitro inhibitory effect of piracetam on the fusion process induced by the two Alzheimer's Beta-amyloid tilted peptides on membrane models :

Four fundamental studies using different tools such as conformational analysis, biophysical methods, NMR and two-hybrid assay were carried out.

They showed the efficacy of piracetam to inhibit the fusion process induced by the two Alzheimer's β-amyloid tilted peptides (29-40 and 29-42) on membrane models.

Results reported that, whatever the amyloid peptide is, preincubation in vitro of piracetam before the addition of each tilted peptide inhibits almost completely the fusion process induced by the two amyloid peptides.

Furthermore, it is interesting to note that , whatever the dose of piracetam is , when piracetam is introduced after incubation of the amyloid peptide with liposomes , piracetam is not able to inhibit the fusion process already in progress .

Two-hybrid assay showed that this effect is neither located in the cytoplasm nor in the nucleus of the cell. This inhibitory effect of piracetam on the fusion process induced by the two amyloid tilted peptides seems to be strictly located at the level of the membrane of the cell.

## Claims

1. The use of a compound of formula I, in racemic or isomeric form, or a pharmaceutically acceptable salt thereof, 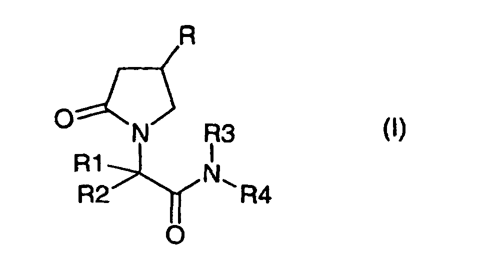 wherein
- R represents hydrogen or hydroxy;
- R¹ and R² represent independently hydrogen or an alkyl group of 1-4 carbon atoms; and
- R³ and R⁴ represent independently hydrogen, an alkyl group of 1-4 carbon atoms or -(CH₂)ₙ-NR⁵R⁶ wherein n is 1, 2 or 3 and R⁵ and R⁶ represent independently hydrogen or an alkyl group of 1-4 carbon atoms;
for the preparation of a medicament to prevent and treat viral disease.

2. The use according to claim 1 wherein the viral disease is selected from Newcastle disease, bovine leukemia, HIV, AIDS, influenza, measles. rubella, hepatitis B, hepatitis C, ebola fever.

3. The use according to claims 1 or 2 wherein R¹, R², R³ and R⁴ are hydrogen.

4. The use according to claims 1 or 2 wherein R¹ is an ethyl, and R², R³ and R⁴ are hydrogen.

5. The use according to claim 4 wherein the compound is in the form of its (R) isomer.

6. The use according to any one of claims 1 to 5 wherein the compound of formula I is in the form of a pharmaceutically acceptable salt.

## Patentansprüche

1. Verwendung einer Verbindung der Formel I in racemischer oder isomerer Form oder eines pharmazeutisch akzeptablen Salzes davon, 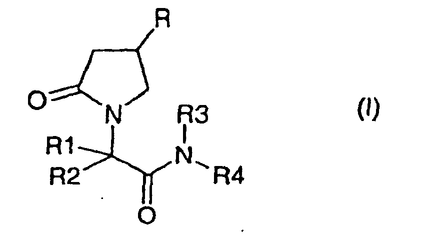 worin
- R Wasserstoff oder Hydroxy darstellt,
- R¹ und R² unabhängig Wasserstoff oder eine Alkylgruppe mit 1 bis 4 Kohlenstoffatomen darstellen, und
- R³ und R⁴ unabhängig Wasserstoff, eine Alkylgruppe mit 1 bis 4 Kohlenstoffatomen oder -(CH₂)ₙ-NR⁵R⁶ darstellen, worin n 1, 2 oder 3 ist, und R⁵ und R⁶ unabhängig Wasserstoff oder eine Alkylgruppe mit 1 bis 4 Kohlenstoffatomen darstellen;
zur Herstellung eines Medikaments, um eine Viruserkrankung vorzubeugen und zu behandeln.

2. Verwendung nach Anspruch 1, wobei die Viruserkrankung aus der Newcastle-Krankheit, Boviner Leukämie, HIV, AIDS, Influenza, Masern, Röteln, Hepatitis B, Hepatitis C, Ebolafieber ausgewählt ist.

3. Verwendung nach den Ansprüchen 1 oder 2, wobei R¹, R², R³ und R⁴ Wasserstoff sind.

4. Verwendung nach den Ansprüchen 1 oder 2, wobei R¹ ein Ethyl ist, und R², R³ und R⁴ Wasserstoff sind.

5. Verwendung nach Anspruch 4, wobei die Verbindung in der Form ihres (R)-Isomers vorliegt.

6. Verwendung nach einem der Ansprüche 1 bis 5, wobei die Verbindung der Formel I in Form eines pharmazeutisch akzeptablen Salzes vorliegt.

## Revendications

1. L'utilisation d'un composé de formule I, sous forme racémique ou isomère, ou de l'un de ses sels pharmaceutiquement acceptable 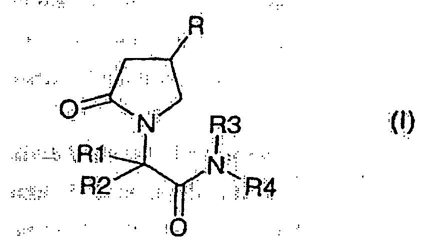 où
- R représente un hydrogène ou un hydroxy ;
- R¹ et R² représentent de manière indépendante un hydrogène ou un groupe alcoyle de 1 à 4 atomes de carbone ; et
- R³ et R⁴ représentent de manière indépendante un hydrogène, un groupe alcoyle de 1 à 4 atomes de carbone ou -(CH₂)ₙ - NR⁵R⁶ où n est 1, 2 ou 3 et R⁵ et R⁶ représentent indépendamment un hydrogène ou un groupe alcoyle de 1 à 4 atomes de carbone ;
pour la préparation d'un médicament qui prévient et traite les maladies virales.

2. L'utilisation selon la revendication 1 dans laquelle la maladie virale est choisie parmi la maladie de Newcastle, la leucémie bovine, le VIH, le SIDA, la grippe, les oreillons, la rubéole, l'hépatite B, l'hépatite C, la fièvre ébola.

3. L'utilisation selon les revendications 1 ou 2 dans laquelle R¹, R², R³ et R⁴ sont hydrogène.

4. L'utilisation selon les revendications 1 ou 2 dans laquelle R¹est un éthyle, et R² R³ et R⁴ sont hydrogène.

5. L'utilisation selon la revendication 4 dans laquelle le composé est sous la forme de son isomère (R).

6. L'utilisation selon n'importe quelle revendication de 1 à 5 dans laquelle le composé de formule I est sous la forme d'un sel pharmaceutiquement acceptable.
